# EUROPEAN PATENT APPLICATION

(11) **EP 0 585 767 A2**
(43) Date of publication of application: **09.03.1994**
(21) Application number: 93113425.8
(22) Date of filing: 23.08.1993
(51) Int. Cl.: C12N 15/55, C12N 15/70, C12N 15/63, C12N 9/20

(54) **Pseudomonas Lipase**

(30) Priority: 27.08.1992 US 936431
(71) Applicant: E.R. SQUIBB & SONS, INC., Princeton New Jersey 08543-4000 (US)
(72) Inventor: Goldberg, Steven L., Basking Ridge NJ. (US); Johnston, Robert M., Whitehouse Station NJ 08889 (US); Cino, Paul M., Bound Brook,NJ 08805 (US)
(74) Representative: Josif, Albert, Dr.-Ing.

(57) **Abstract**

Nucleic acid sequences, particularly DNA sequences, coding for all or part of a lipase of Pseudomonas sp. ATCC 21808, wherein said lipase has the amino acid sequence encoded by the nucleotide sequence of the Sacl/Hindlll insert of plasmid pSG312 deposited with the American Type Culture Collection, expression vectors containing the DNA sequences, host cells containing the expression vectors, and methods utilizing these materials. The invention also concerns polypeptide molecules comprising all or part of a lipase of Pseudomonas sp. ATCC 21808, wherein said lipase has the amino acid sequence encoded by the nucleotide sequence of the Sacl/Hindlll insert of plasmid pSG312 deposited with the American Type Culture Collection, and methods for producing these polypeptides molecules.

## Description

Lipases are a large group of enzymes which are found in many different species. In nature, these enzymes are primarily responsible for catalyzing the conversion of triglycerides to fatty acids. Lipases have been isolated from many different species. For example International Patent Application, Publication No. WO 91/00908, European Patent Application, Publication No. 0 407 225 A1, European Patent Application, Publication No. 0 443 063 A1 and U.S. Patent No. 5,063,160 all describe the isolation of lipase from various Pseudomonas species. However, there is still a need for lipases which may have more favorable characteristics than those already isolated.

The present invention concerns an isolated nucleic acid molecule comprising a nucleic acid sequence coding for all or part of a lipase of Pseudomonas sp. ATCC 21808 (hereinafter the "subject lipase"), wherein said lipase has the amino acid sequence encoded by the nucleotide sequence of the SacI/HindIII insert of plasmid pSG312 deposited with the American Type Culture Collection (hereinafter the "subject insert"). Preferably, the nucleic acid molecule is a DNA (deoxyribonucleic acid) molecule, and the nucleic acid sequence is a DNA sequence. Further preferred is a DNA sequence having all or part of the subject insert.

The present invention further concerns expression vectors comprising a DNA sequence coding for all or part of the subject lipase.

The present invention also concerns prokaryotic or eukaryotic host cells containing an expression vector which comprises a DNA sequence coding for all or part of the subject lipase.

The present invention additionally concerns polypeptide molecules comprising all or part of the subject lipase.

### FIGURE 1

a. Partial restriction map of Pseudomonas DNA in pSG300. Relative locations of restriction sites are given in base pairs.
b. Deletion analysis of insert DNA in pSG300. Lipolytic activity of the various deletions are indicated. Blank spaces indicate deleted regions.

The present invention concerns an isolated nucleic acid molecule comprising a nucleic acid sequence coding for all or part of the subject lipase. Preferably, the nucleic acid molecule is a DNA molecule and the nucleic acid sequence is a DNA sequence. Further preferred is a DNA sequence having all or part of the nucleotide sequence of the subject insert or a DNA sequence complementary to this DNA sequence. In the case of a nucleotide sequence (e.g., a DNA sequence) coding for part of the subject lipase, it is preferred that the nucleotide sequence be at least about 15 sequential nucleotides in length, more preferably at least about 20 or 30 sequential nucleotides in length.

The DNA sequences of the present invention can be isolated from a variety of sources, although the presently preferred sequence has been isolated from genomic DNA obtained from Pseudomonas species ATCC 21808.

The DNA sequences of the present invention can be obtained using various methods well-known to those of ordinary skill in the art. At least three alternative principal methods may be employed:
(1) the isolation of a double-stranded DNA sequence from genomic DNA which contains the sequence;
(2) the chemical synthesis of the DNA sequence; and
(3) the synthesis of the DNA sequence by polymerase chain reaction (PCR).

In the first approach, a genomic DNA library can be screened in order to identify a DNA sequence coding for all or part of the subject lipase.

Various techniques can be used to screen the genomic DNA library. For example, labeled single stranded DNA probe sequences complementary to or duplicating a sequence present in the target genomic DNA coding for all or part of the subject lipase can be employed in DNA/DNA hybridization procedures carried out on cloned copies of the genomic DNA which have been denatured to single stranded form.

A genomic DNA library can also be screened for a genomic DNA coding for all or part of the subject lipase.

In one typical screening method suitable for hybridization techniques, the genomic DNA library, which is usually contained in a bacteriophage vector or plasmid such as λgt11 or pUC19, respectively, is first spread out on agarose plates, and then the clones (plaques or colonies) are transferred to filter membranes, for example, nitrocellulose membranes. A DNA probe can then be hybridized to the clones to identify those clones containing the genomic DNA coding for all or part of the subject lipase.

In the second approach, the DNA sequences of the present invention coding for all or part of the subject lipase can be chemically synthesized. For example, the DNA sequence coding for the subject lipase can be synthesized as a series of 50-100 base oligonucleotides that can then be sequentially ligated (via appropriate terminal restriction sites) so as to form the correct linear sequence of nucleotides.

In the third approach, the DNA sequences of the present invention coding for all or part of the subject lipase can be synthesized using polymerase chain reaction (PCR) techniques. Briefly, pairs of synthetic DNA oligonucleotides generally at least 15 bases in length (PCR primers) that hybridize to opposite strands of the target DNA sequence are used to enzymatically amplify the intervening region of DNA on the target sequence. Repeated cycles of heat denaturation of the template, annealing of the primers and extension of the 3'-termini of the annealed primers with a DNA polymerase results in amplification of the segment defined by the 5' ends of the PCR primers. See White, T.J. et al., Trends Genet. 5, 185-189(1989).

The DNA sequences of the present invention can be used in a variety of ways in accordance with the present invention. For example, they can be used as DNA probes to screen other genomic DNA libraries so as to select by hybridization other DNA sequences that code for related proteins.

The DNA sequences of the present invention coding for all or part of the subject lipase can also be modified (i.e., mutated) to prepare various mutations. Such mutations may be either degenerate, i.e., the mutation does not change the amino acid sequence encoded by the mutated codon, or non-degenerate, i.e., the mutation changes the amino acid sequence encoded by the mutated codon. These modified DNA sequences may be prepared, for example, by mutating the DNA sequence encoding the subject lipase so that the mutation results in the deletion, substitution, insertion, inversion or addition of one or more amino acids in the encoded polypeptide using various methods known in the art. For example, the methods of site-directed mutagenesis described in Taylor, J. W. et al., Nucl. Acids Res. 13, 8749-8764 (1985) and Kunkel, J. A., Proc. Natl. Acad. Sci. USA 82, 482-492 (1985) may be employed. In addition, kits for site-directed mutagenesis may be purchased from commercial vendors. For example, a kit for performing site-directed mutagenesis may be purchased from Amersham Corp. (Arlington Heights, IL). In addition, disruption, deletion and truncation methods as described in Sayers, J. R. et al., Nucl. Acids Res. 16, 791-800(1988) may also be employed. Both degenerate and non-degenerate mutations may be advantageous in producing or using the polypeptides of the present invention. For example, these mutations may modify the function of the protein (eg., result in higher or lower activity), permit higher levels of production, easier purification, or provide additional restriction endonuclease recognition sites. All such modified DNA and polypeptide molecules are included within the scope of the present invention.

As used in the present application, the term "modified", when referring to a nucleotide or polypeptide sequence, means a nucleotide or polypeptide sequence which differs from the wild-type sequence found in nature.

The present invention further concerns expression vectors comprising a DNA sequence coding for all or part of the subject lipase. The expression vectors preferably contain all or part of the DNA sequence having the nucleotide sequence of the subject insert. The expression vectors in general contain one or more regulatory DNA sequences operatively linked to the DNA sequence coding for all or part the subject lipase. As used in this context, the term "operatively linked" means that the regulatory DNA sequences are capable of directing the replication and/or the expression of the DNA sequence coding for all or part of the subject lipase.

Expression vectors of utility in the present invention are often in the form of "plasmids", which refer to circular double stranded DNA loops which, in their vector form, are not bound to the chromosome. However, the invention is intended to include such other forms of expression vectors which serve equivalent functions and which become known in the art subsequently hereto.

Expression vectors useful in the present invention typically contain an origin of replication, a promoter located in front of (i.e., upstream of) the coding DNA sequence, the DNA sequence coding for all or part of the subject lipase, and transcription termination sequences. The expression vectors may also include other DNA sequences known in the art; for example, stability leader sequences which provide for stability of the expression product, secretory leader sequences which provide for secretion of the expression product, sequences which allow expression of the structural gene to be modulated (e.g., by the presence or absence of nutrients or other inducers in the growth medium), sequences which are capable of providing phenotypic selection in transformed host cells, sequences which provide sites for cleavage by restriction endonucleases, and sequences which allow expression in various types of hosts, including but not limited to prokaryotes, yeasts, fungi, plants and higher eukaryotes.

The characteristics of the actual expression vector used must be compatible with the host cell employed. For example, when cloning in a mammalian cell system, the expression vector should contain promoters isolated from the genomic DNA of mammalian cells, (e.g., mouse metallothionien promoter), or from viruses that grow in these cells (e.g., vaccinia virus 7.5 K promoter). An expression vector as contemplated by the present invention is at least capable of directing the replication, and preferably the expression, of the DNA sequences of the present invention. Suitable origins of replication include, for example, pMB1, ColE1, and SV40 ori. Suitable promoters include, for example, Plac, Ptac, HCMV, and Pₜₖ. Suitable termination sequences include, for example, the trp, T7 and, SV40 terminators. As selectable markers, chloramphenicol, ampicillin, neomycin, and hygromycin resistance can be employed. All of these materials are known in the art and are commercially available.

Suitable commercially available expression vectors into which the DNA sequences of the present invention may be inserted include pBR322, pUC19 or pBC-SK⁺.

Suitable expression vectors containing the desired coding and control sequences may be constructed using standard recombinant DNA techniques known in the art, many of which are described in Sambrook, et al., Molecular Cloning: A Laboratory Manual, Second Edition, Cold Spring Harbor Laboratory, Cold Spring Habor, NY (1989).

The present invention additionally concerns host cells containing an expression vector which comprises a DNA sequence coding for all or part of the subject lipase. The host cells preferably contain an expression vector which comprises all or part of the DNA sequence having the nucleotide sequence of the subject insert. In general, the host cells contain an expression vector comprising one or more regulatory DNA sequences capable of directing the replication and/or the expression of and operatively linked to a DNA sequence coding for all or part of the subject lipase. Suitable host cells include both prokaryotic and eukaryotic cells. Suitable prokaryotic host cells include, for example, E. coli strains BL21, W3110, JM83, and XL-1 Blue. Suitable eukaryotic host cells include, for example, COS, CHO, and CV-1.

Expression vectors may be introduced into host cells by various methods known in the art. For example, transfection of mammalian host cells with expression vectors can be carried out by the calcium phosphate precipitation method. Transformation of E. coli is commonly performed after treatment with calcium chloride. However, other methods for introducing expression vectors into host cells, for example, electroporation, liposomal fusion, nuclear injection, and viral or phage infection can also be employed.

Once an expression vector has been introduced into an appropriate host cell, the host cell may be cultured under conditions permitting expression of large amounts of the desired polypeptide, in this case a polypeptide molecule comprising all or part of the subject lipase. Such polypeptides are particularly useful in enzymatic processes for the resolution of enantiomeric mixtures of compounds as described herein below and in U.S. Patent Application Serial No. 07/822,015 filed January 15,1992 and entitled "Enzymatic Processes for Resolution of Enantiomeric Mixtures of Compounds Useful as Intermediates in the Preparation of Taxanes", the specification of which is incorporated herein by reference.

Host cells containing an expression vector which contains a DNA sequence coding for the subject lipase may be identified by one or more of the following five general approaches: (a) DNA-DNA hybridization; (b) the presence or absence of marker gene functions; (c) assessing the level of transcription as measured by the production of mRNA transcripts encoding the subject lipase in the host cell; (d) detection of the gene product immunologically; and (e) enzyme assay.

In the first approach, the presence of a DNA sequence coding for all or part of the subject lipase can be detected by DNA-DNA or RNA-DNA hybridization using probes complementary to the DNA sequence.

In the second approach, the recombinant expression vector host system can be identified and selected based upon the presence or absence of certain marker gene functions (e.g., thymidine kinase activity, resistance to antibiotics, etc.). A marker gene can be placed in the same plasmid as the DNA sequence coding for all or part of the subject lipase under the regulation of the same or a different promoter used to regulate the sequence encoding the subject lipase. Expression of the marker gene in response to induction or selection indicates expression of the DNA sequence coding for all or part of the subject lipase.

In the third approach, the production of mRNA transcripts encoding the subject lipase can be assessed by hybridization assays. For example, polyadenylated RNA can be isolated and analyzed by Northern blotting or nuclease protection assay using a probe complementary to the RNA sequence. Alternatively, the total nucleic acids of the host cell may be extracted and assayed for hybridization to such probes.

In the fourth approach, the expression of all or part of the high molecular weight subunit of the subject lipase can be assessed immunologically, for example, by Western blotting.

In the fifth approach, expression of the high molecular weight subunit of the subject lipase can be measured by assaying for lipase activity using known methods. For example, the assay described hereinbelow may be employed.

The DNA sequences of expression vectors, plasmids or DNA molecules of the present invention may be determined by various methods known in the art. For example, the dideoxy chain termination method as described in Sanger et al., Proc. Natl. Acad. Sci. USA 74, 5463-5467 (1977), or the Maxam-Gilbert method as described in Proc. Natl. Acad. Sci. USA 74, 560-564 (1977) may be employed.

It should, of course, be understood that not all expression vectors and DNA regulatory sequences will function equally well to express the DNA sequences of the present invention. Neither will all host cells function equally well with the same expression system. However, one of ordinary skill in the art may make a selection among expression vectors, DNA regulatory sequences, and host cells using the guidance provided herein without undue experimentation and without departing from the scope of the present invention.

The present invention further concerns polypeptide molecules comprising all or part of the subject lipase. In the case of polypeptide molecules comprising part of the subject lipase, it is preferred that polypeptide molecules be at least about 5 to 8 sequential amino acids in length, more preferably at least about 15 to 20 sequential amino acids in length. Also preferred are polypeptides at least about 180 sequential amino acids in length, which may approximate the size of a structural domain within the protein.

The polypeptides of the present invention may be obtained by synthetic means, i.e., chemical synthesis of the polypeptide from its component amino acids, by methods known to those of ordinary skill in the art. For example, the solid phase procedure described by Houghton et al., Proc. Natl. Acad. Sci. 82, 5131-5135 (1985) may be employed. It is preferred that the polypeptides be obtained by production in prokaryotic or eukaryotic host cells expressing a DNA sequence coding for all or part of the subject lipase or by in vitro translation of the mRNA encoded by a DNA sequence coding for all or part of the subject lipase. For example, plasmid pSG312 deposited with the American Type Culture Collection may be used to transform a suitable host cell. The recombinant host cell may then be cultured to produce the subject lipase. Techniques for the production of polypeptides by these means are known in the art, and are described herein.

The polypeptides produced in this manner may then be isolated and purified using various protein purification techniques. For example, chromatographic procedures such as ion exchange chromatography, gel filtration chromatography and immunoaffinity chromatography may be employed.

The polypeptides of the present invention may be used in a wide variety of ways. For example, the polypeptides may be used to prepare in a known manner polyclonal or monoclonal antibodies capable of binding the polypeptides. These antibodies may in turn be used for the detection of the polypeptides of the present invention in a sample, for example, a cell sample, using immunoassay techniques, for example, radioimmunoassay or enzyme immunoassay. The antibodies may also be used in affinity chromatography for purifying the polypeptides of the present invention and isolating them from various sources.

Due to the degeneracy of the genetic code, other DNA sequences which encode the subject lipase may be used for the production of the polypeptides of the present invention. In addition, it will be understood that allelic variations of these DNA and amino acid sequences naturally exist, or may be intentionally introduced using methods known in the art. These variations may be demonstrated by one or more amino acid differences in the overall sequence, or by deletions, substitutions, insertions, inversions or additional of one or more amino acids in said sequence. Such amino acid substitutions may be made, for example, on the basis of similarity in polarity, charge, solubility, hydrophobicity, hydrophilicity and/or the amphiphathic nature of the residues involved. For example, negatively charged amino acids include aspartic acid and glutamic acid; positively charged amino acids include lysine and arginine; amino acids with uncharged polar head groups or nonpolar head groups having similar hydrophilicity values include the following: leucine, isoleucine, valine, glycine, alanine; asparagine, glutamine; serine, threonine; phenylalanine, tyrosine. Other contemplated variations include salts and esters of the aforementioned polypeptides, as well as precursors of the aforementioned polypeptides, for example, precursors having N-terminal substituents such as methionine, N-formylmethionine and leader sequences. All such variations are included within the scope of the present invention.

The following examples are further illustrative of the present invention. These examples are not intended to limit the scope of the present invention, and provide further understanding of the invention.

### EXAMPLES

### A. Construction of a Pseudomonas sp. ATCC 21808 DNA gene bank

### 1. Isolation of genomic DNA from Pseudomonas sp. ATCC 21808 (American Type Culture Collection, Rockville, MD)

Genomic DNA was isolated from Pseudomonas sp. ATCC 21808, which was identified and characterized at Amano Pharmaceutical Co., Ltd., Nagoya, Japan. The exact species of this Pseudomonas strain is unknown. Details on the organism and lipase synthesized by it can be found in U.S. Patent No. 3,875,007, entitled "Lipid Metabolism Improving and Anti-Atheromatic Agent".

Pseudomonas sp. ATCC 21808 was grown in Luria broth, which consists of 10 grams Bacto-tryptone (Difco Laboratories, Detroit, MI), 5 grams of Bacto yeast extract (Difco Laboratories) and 5 grams of sodium chloride (Sigma Chemical Co., St. Louis, MO), pH 7.0, per liter. Cultures were incubated with shaking at 37°C for 24 hours. Cells were pelleted at 8,000 x g for 5 minutes and resuspended in 20 ml of 0.3 M sucrose (Sigma Chemical Co.), 0.025 M Tris-HCl, pH 8.0 (Sigma Chemical Co.), 0.025 M ethylenediaminetetraacetic acid (EDTA; Sigma Chemical Co.) containing 5 mg/mL lysozyme (Sigma Chemical Co.) per 100 mL starting culture. After incubation at 22°C for 10 minutes, lysis of protoplasts was achieved by addition of 1.0 mL of a 20% sodium dodecyl sulfate (SDS; Sigma Chemical Co.) solution, followed by rapid inversion of the tube until the sample was viscous and had cleared. Cellular proteins were hydrolyzed by addition of 1.0 mL of a 20 mg/mL Proteinase K solution (Bethesda Research Laboratories, Gaithersburg, MD) and shaking the samples slowly for 1 hour at 37°C. Nucleic acids were isolated by addition of an equal volume of phenol/chloroform/isoamyl alcohol (25:24:1) equilibrated with TE buffer (0.01 M Tris-HCl, pH 8.0, 0.001 M EDTA). After gentle shaking for 30 minutes at 22°C, the sample was centrifuged at 8,000 x g for 10 minutes and the viscous upper (aqueous) phase was carefully removed using a sterile wide-tip pipette. An equal volume of chloroform (Fisher Scientific, Fair Lawn, NJ) was added and thoroughly mixed. The preparation was centrifuged at 10,000 x g for 10 minutes and the aqueous layer transferred to a sterile 50 mL disposable tube.

Nucleic acids were precipitated by the addition of 0.1 volume of 3 M sodium acetate (Sigma Chemical Co.) and 2 volumes of anhydrous (95%) ethanol (International Biotechnologies Inc., New Haven, CT). The stringy mass appearing after gentle inversion was removed using a sterile bent Pasteur pipette. Salts were washed off the pellet by rinsing it in 70%, then 95%, ethanol, and allowed to air dry in a fresh 50 mL disposable tube.

To further remove any RNA and protein from the chromosomal DNA, the pellet was resuspended in 17.6 ml TE buffer to which 19.2 grams cesium chloride (Bethesda Research Laboratories, Gaithersburg, MD) and 0.8 mL of 10 mg/mL ethidium bromide (EtBr; Sigma Chemical Co.) was added. This solution was distributed between four 5.1 mL ultracentrifuge tubes and centrifuged at 60,000 rpm at 20°C for 24 hours in a Beckman VTi65 rotor (Beckman Instruments, Inc., Palo Alto, CA). After exposure to 320 nm ultraviolet light, the single fluorescing band was removed by puncturing the tubes with an 18 gauge needle and drawing the sample out with a 3 cc syringe. Ethidium bromide was extracted by addition of an equal volume of cesium chloridesaturated isoamyl alcohol (Fisher Scientic, Fair Lawn, NJ) in a 15-mL plastic tube. The mixture was gently inverted several times, following which the aqueous and organic phases were permitted to separate for 2 minutes. The upper, organic layer was removed and the procedure repeated until no pink color could be seen in the aqueous phase. Purified Pseudomonas DNA was precipitated by the addition of 2 volumes of sterile distilled water and 6 volumes of 95% ethanol. DNA was again recovered with a bent Pasteur pipette, washed with 70% and 95% ethanol, and resuspended in 0.5 mL TE buffer. A 1:100 dilution of the DNA in TE buffer was used to spectrophotometrically calculate the concentration of the original solution using the formula OD₂₆₀ₙₘ₋₃₂₀ₙₘ x 100 x 50 (where 1 OD₂₆₀ₙₘ₋₃₂₀ₙₘ unit of double-stranded DNA = 50 µg/mL).

### 2. Isolation of 2-6 Kilobase Size Fragments of Pseudomonas sp. ATCC 21808 DNA

Purified Pseudomonas sp. ATCC 21808 chromosomal DNA (50 µg) and 12.5 units (U) of restriction endonuclease Sau 3A1 (Bethesda Research Laboratories, Gaithersburg, MD) were mixed in a reaction volume of 0.5 mL containing 0.02 M Trishydrochloride pH 7.4, 0.005 M magnesium chloride, and 0.05 M potassium chloride. After incubation at 37°C for 30 minutes, the reaction was terminated and proteins removed from the sample by addition of an equal volume of TE-buffered phenol/chloroform/isoamyl alcohol (25:24:1, v/v/v). The sample was centrifuged for 1 minute at 13,000 x g in a Marathon 13K/M microcentrifuge (Fisher Scientific, Pittsburgh, PA) and the upper aqueous phase retained. A small aliquot of the preparation was analyzed by electrophoresis in a 0.7% agarose gel with TAE buffer (consisting of 0.04 M Trizma base, 0.02 M acetate and 0.001 M EDTA, pH 8.3; Sigma Chemical Co.) and 0.5 µg/mL ethidium bromide for 1 hour at 100 volts. The remaining sample was electrophoresed in a preparative 0.7% agarose gel for 18 hours at 10 volts. DNA fragments from 2-6 kilobases in length were excised from the gel and electroeluted at 8 mA for 3 hours in TAE buffer using a Fisher Biotech Electrophoresis Gel Eluter System (Fisher Scientific, Pittsburgh, PA). DNA was precipitated by addition of 0.5 volume of 7.5 M ammonium acetate (Sigma Chemical Co.) and 2 volumes of 95% ethanol. After incubating at -70°C for 5 minutes, the precipitated DNA was collected by centrifugation for 5 minutes at 13,000 x g in a microcentrifuge. The pellet was washed once with 0.2 ml 70% ethanol, air dried, and resuspended in TE buffer to a final concentration of 100 µg/mL.

### 3. Ligation of Pseudomonas sp. ATCC 21808 DNA to plasmid vector pBC-SK⁺

Plasmid pBC-SK⁺ (Stratagene, Inc., La Jolla, CA) is a modification of pUC19 (Yanisch-Perron et al., Gene 33 103-109 [1985]). pBC-SK⁺ is 3401 base pairs (bp) in size, contains many unique restriction endonuclease sites, encodes resistance to chloramphenicol, and exists at high copy number in Escherichia coli.

pBC-SK⁺ was cleaved with the restriction enzyme BamHl in a 0.05 ml reaction volume consisting of 0.5 µg plasmid DNA, 2.5 U BamHl (Bethesda Research Laboratories, Gaithersburg, MD), 0.05 M Tris-HCl pH 8.0, 0.01 M MgCl₂, and 0.1 M NaCl. The sample was incubated at 37°C for 1.5 hours and extracted once with an equal volume of phenol/chloroform/isoamyl alcohol (25:24:1, v/v/v). After centrifugation for 1 minute at 13,000 x g in a microcentrifuge, the upper phase was removed and the DNA was precipitated by addition of 0.5 volume of 7.5 M NH₄Cl and 2 volumes of 95% ethanol. The sample was held at -70°C for 5 minutes and DNA pelleted by centrifugation at 13,000 x g for 5 minutes. The sample was washed once with 0.2 mL 70% ethanol and air-dried before resuspending in TE buffer at 100 µg/mL.

The size-fractioned Pseudomonas sp. ATCC 21808 DNA (0.5 µg) and BamHl-digested pBC-SK⁺ plasmid DNA (0.1 µg) were ligated in a 0.03 mL reaction volume which contained 0.05 M Tris-HCl (pH 7.6), 0.01 M MgCl₂, 0.001 M adenosine triphosphate (ATP), 0.001 M dithiothreitol (DTT), 5% (w/v) polyethylene glycol 8000 (PEG 8000), and 1 U of T4 DNA ligase (Bethesda Research Laboratories, Gaithersburg, MD). The reaction proceeded at 4°C for 18 hours. One unit of ligase catalyzes the exchange of 1 nmol ³²P-labeled pyrophosphate into ATP in 20 minutes at 37°C in a 0.1 mL reaction volume with 0.066 M Tris-HCl (pH 7.6), 0.0066 M MgCl₂, 0.01 M DTT, 0.006 mM ATP and 0.0033 mM ³²P-labeled pyrophosphate.

### 4. Transformation of Escherichia coli with ligated Pseudomonas sp. ATCC 21808-pBC-SK⁺ DNA

Competent XL-1 Blue E. coli cells (Stratagene, Inc., La Jolla, CA) were prepared according to the procedure of Chung et al., Proc. Natl. Acad. Sci. USA 86, 2172-2175 (1989). In summary, a single colony of XL-1 Blue was inoculated into a tube containing 5 mL of Luria broth supplemented with 12.5 mg/mL tetracycline (Tc). The culture was grown at 30°C overnight at 250 rpm on a rotary shaker. A 1:100 dilution of the culture into fresh Luria both was made and incubated at 30°C, 250 rpm on a rotary shaker until the A₆₀₀ reached 0.3-0.4. The cells were decanted into a chilled tube and centrifuged at 8,000 x g for 5 minutes at 4°C. The pellet was gently resuspended in TSS buffer, consisting of Luria broth supplemented with 20% (w/v) PEG-3350, 0.1 M MgCl₂, and 10% (v/v) dimethyl sulfoxide, pH 6.5.

A 200 µL aliquot of competent XL-1 Blue cells was mixed with 1.0 µL of the ligated DNA in a pre-chilled Falcon 2059 polypropylene tube (Becton Dickenson and Co., Lincoln Park, NJ). The tube was held on ice for 30 minutes, following which 0.8 mL of SOC medium, which is 0.5% yeast extract, 2.0% tryptone, 10 mM NaCl, 2.5 mM KCl, 10 mM MgCl₂, 10 mM MgSO₄, and 20 mM glucose, was added. The sample was incubated at 225 rpm at 30°C on a rotary shaker for 2 hours. The entire sample was transferred to a microfuge tube and centrifuged at 13,000 x g for 1 minute. The cell pellet was resuspended in 100 µL of SOC medium.

### 5. Identification of recombinant of E. coli expressing Pseudomonas sp. ATCC 21808 lipase

Transformed E. coli XL-1 Blue cells were spread onto Spirit Blue (SB) agar medium (Difco Laboratories, Detroit, MI) supplemented with 3% (v/v) Difco Lipase Reagent (Difco Laboratories, Detroit, MI) and 30 µg/mL chloramphenicol (Cm; Sigma Chemical Co.). The plates were incubated at 37°C for 48 hours and examined for zones of lipolytic activity around individual colonies. One such colony was isolated and restreaked onto a fresh SB Cm plate, then incubated as described above. All colonies maintained the lipolytic phenotype, whereas no lipolytic colonies were seen using a control transformation with plasmid vector pBC-Sk⁺.

### B. Characterization of the Pseudomonas sp. ATCC 21808 lipase and its corresponding DNA cloned into E. coli

### 1. Estimation of the molecular weight of lipase produced by lipolytic XL-1 Blue transformants.

E. coli cells expressing the Pseudomonas sp. ATCC 21808 lipase were grown in Luria broth containing Cm for 18 hours at 37°C, 250 rpm, on a rotary shaker. The 0D₆₀₀ was recorded and the cells were centrifuged at 13,000 x g for 2 minutes. The pellet was first resuspended in TE + 5 mg/mL lysozyme, after which an equal volume of 2x gel loading buffer (125 mM Tris-HCl, pH 6.8., 20% [v/v] glycerol [Sigma Chemical Co.], 5% [v/v]β -mercaptoethanol [Sigma], and 0.025% [w/v] Bromophenol Blue [Sigma]) was added. Up to 30 µL of the preparation was electrophoresed on a 10-20% sodium dodecyl sulfate-polyacylamide mini-gel (Integrated Separation Systems, Hyde Park, MA) at 50 mA for 1.5 hours. The gel was removed from the apparatus and gently shaken in a 2.5% Triton X-100 solution for 1 hour, then rinsed five times in distilled water. The gel was transferred to the bottom of a 150 mm petri plate and overlayed with molten SB Cm medium cooled to 50°C. After 24-48 hours at 37°C, a band of clearing in the lipid emulsion appeared at the location of lipolytic activity in the polyacrylamide gel.

The molecular weight of the recombinant lipase was the same as that of a commercially-available lipase isolated from Pseudomonas sp. ATCC 21808 (Amano Pharmaceutical Co., Ltd., Nagoya, Japan), or 33,000 daltons, included as a control in the polyacrylamide gel. No activity was present using lysates from XL-1 Blue (pBC-SK⁺) or from heat-inactivated material (100°C, 15 minutes) from the recombinant E. coli isolate.

### 2. Isolation and restriction analysis of the recombinant plasmid expressing the Pseudomonas sp. ATCC 21808 lipase

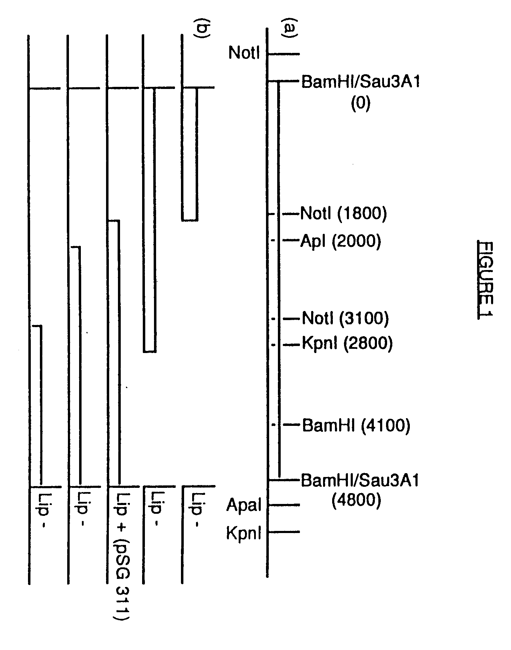

Plasmid DNA from the XL-1 Blue transformant which demonstrated lipase activity was isolated from 50 mL LB Cm cultures using a kit obtained from Qiagen, Inc. (Chatsworth, CA). The purified DNA pellet was resuspended in TE buffer to a final concentration of 1 mg/mL. The plasmid DNA was analyzed and a restriction map created as shown in Figure 1 using enzymes and buffers provided by the manufacturer (Bethesda Research Laboratories, Gaithersburg, MD). A DNA insert of 4800 bp was present in pBC-SK⁺ and the recombinant plasmid renamed pSG300.

### 3. Subcloning and deletion analysis of recombinant plasmid pSG300

Plasmid pSG300 was cleaved with the restriction enzymes Apal, Kpnl, Xhol and Notl (Bethesda Research Laboratories, Gaithersburg, MD). The samples were electrophoresed through a 0.7% TAE-EtBr-agarose gel for 1.5 hours at 100 volts. All fragments were isolated from the gel and purified using the GeneClean Kit. Internal fragments of Pseudomonas sp ATCC 21808 DNA, identified on the basis of the pSG300 restriction map, were ligated to pBC-SK⁺ digested with the identical enzyme. Those fragments containing the pBC-SK⁺ vector and a part of the Pseudomonas sp. ATCC 21808 DNA insert were self-ligated. DNAs from the ligation reactions were transformed into competent XL-1 Blue cells as previously described. After 48 hours at 37°C, lipolytic and non-lipolytic colonies on the SB Cm plates were purified once on the same medium. Plasmid DNA from these isolates was prepared and analyzed by restriction endonuclease digestion, followed by agarose gel electrophoresis.

The Apal and Xhol digestions yielded non-lipolytic transformants except for instances where the entire 4800 bp insert had been fortuitously religated. However, a Notl fragment of 3000 bp was able to confer lipolytic activity to XL-1 Blue transformants. The plasmid containing this insert was named pSG310.

To further define the region required for lipolytic activity, unidirectional deletions of both ends of the cloned DNA present in pSG310 were performed using Exonuclease lll (Exolll) and Mung Bean nuclease. All materials were supplied in a kit purchased from Stratagene, Inc., La Jolla, CA, except for restriction enzymes and buffers, which were obtained from Bethesda Research Laboratories, Gaithersburg, MD.

For deletion of the "left" end of the insert, 25 µg of purified pSG310 was first cleaved with 100 U of Sstl in a 100 µL reaction volume for 2 hours at 37°C. The sample was extracted with an equal volume of phenol: CHCl₃: isoamyl alcohol and ethanolprecipitated. The DNA was pelleted at 13,000 x g for 5 minutes, washed with 0.2 mL 70% Ethanol, and resuspended in sterile distilled water. A second digest using Xbal was carried out as described above and the pelleted DNA dissolved in 25 mL of sterile distilled water.

For deletion of the "right" end of the insert, 25 µg of purified pSG310 DNA was digested with 100 U of Hindlll. Since the 5' overhangs left after restriction by Hindlll are recognized by Exolll, they were protected from the nuclease by filling in using modified deoxyribonucleotides. Following cleavage, 360 µL of sterile distilled water, 40 µL of 10x Hindlll buffer, 2 µL of a 1 mM thiodeoxyribonucleotide mixture, and 50 of Klenow DNA polymerase were added. After 10 minutes at 22°C, the reaction was terminated by addition of an equal volume of phenol: CHCl₃: isoamyl alcohol, then precipitated with ethanol. The DNA was pelleted by centrifugation for 5 minutes at 13,000 x g and resuspended in sterile distilled water. The sample was subjected to Smal digestion as previously described and the pelleted DNA dissolved in 25 µL of sterile distilled water.

The restricted DNA preparations were then treated with Exolll. 2x Exolll buffer (62.5 µL; 100 mM Tris-HCl, pH 8.0,10 mM MgCl₂, 20 µg/mL transfer RNA), 12.5 µL 100 mM β-mercaptoethanol, 5.0 µL (500 U) of Exonuclease lll, and 25 µL of additional sterile distilled water were combined in a 1.5 mL microfuge tube and incubated at 35°C. At regular time intervals, 25 µL aliquots of the reaction were removed and transferred to a separate tube containing 20 µL of 10x Mung Bean nuclease buffer (300 mM sodium acetate [Sigma Chemical Co., St. Louis], pH 5.0, 500 mM NaCl, 10 mM zinc chloride [Sigma], and 50% [v/v] glycerol) to terminate the Exolll reaction. The samples were heated at 68°C for 15 minutes and chilled on ice. Mung Bean nuclease (15 U) was added and the tubes incubated at 30°C for 30 minutes to create blunt ends. DNA was extracted with phenol: CHCl₃: isoamyl alcohol, precipitated, centrifuged, and resuspended in 10 µL of sterile distilled water and 1 µL of DNA loading dye. The preparation was electrophoresed through a 0.7% TAE-ethidium bromide-agarose gel for 3 hours at 100 volts. The DNA band of a lower size than the full-length control was excised from the gel and extracted using GeneClean. The deleted, linear plasmid DNA was recircularized using T4 DNA ligase at a nucleic acid concentration of 20 µg/mL and transformed into competent XL-1 Blue cells. Lipolytic and non-lipolytic colonies were observed on SB Cm agar medium after 24-36 hours at 37°C.

The extent of any deletions was determined by subjecting mini-plasmid preparations of the Exolll/Mung Bean nuclease-treated pSG310 to Kpnl and/or Apal restriction digestion. A summary of the results obtained is presented in Figure 1, which indicate that a region of 2200 bp in length is ncessary for lipolytic activity in E. coli. Since only about 1000 bp of DNA is needed to encode a protein of 33,000 daltons the function of an "activator" gene similar to that associated with other Pseudomonas lipases (Irzumi et al., Agric. Bio. Chem. 55, 2349-2357 [1991]; Jorgensen et al., J. Bacteriol. 173, 559-567 [1991]) may also be required for full activity of the cloned Pseudomonas sp. ATCC 21808 lipase.

### C. Expression of the Pseudomonas sp. ATCC 21808 lipase in E. coli

### 1. Construction of plasmid pSG312

Plasmids pSG310 and pUC19 (2 µg each) were cleaved with 6.6 U of restriction endonucleases Sacl and Hindlll. Following electrophoresis through a 0.7% TAE-EtBr-agarose gel for 1.5 hours at 100 volts, the fragment from pSG310 of about 3000 bp encompassing the cloned lipase gene and the 2691 bp band of pUC19 were excised and purified using GeneClean. The two fragments were then ligated together using 500 ng and 100 ng of DNA, respectively, in a total volume of 30 µL. Ligation proceeded for 2 hours at room temperature. A 2 µL aliquot was used to transform competent XL-1 Blue cells, which were spread onto SB Cm agar medium. After 24 hours at 37°C, lipolytic colonies were visible and purified twice on the same medium. The plasmid obtained from the above ligation/transformation was named pSG311.

The ampicillin-resistance gene present on pSG311 was subsequently replaced with the chloramphenicol-resistance factor. Two µg of pSG311 was digested with 5 U of the restriction enzyme Pvull in a total volume of 20 µL. The sample was electrophoresed and the fragment of about 3260 bp isolated from the agarose gel as previously described. Plasmid pBC-SK⁺ (6 µg) was partially cleaved with Pvull using 3 U of enzyme in a 20 µL reaction volume at 37°C for 30 minutes. The 2952-bp fragment, encompassing the Cm-resistance gene and plasmid replicating elements, was isolated from a 0.7% agarose gel following electrophoresis is using GeneClean. A ligation using 250 ng of Pvull-digested pSG311 and 100 ng of the partially-cleaved pBC-SK⁺ DNA in a 10 µL final volume was performed. Competent XL-1 Blue cells were transformed with 2 µL of the reaction and spread onto SB Cm agar medium. Strongly lipolytic colonies appeared after 24 hours at 37°C. Restriction analysis confirmed the presence of the a lipaseencoding DNA fragment in pBC-SK⁺. This construct was named pSG312.

### 2. Construction of plasmid pSG370

Expression vector pKK223-3, which possesses the very strong, inducible tac promoter, was purchased from Pharmacia/LKB (Piscataway, NJ). An aliquot of this plasmid (2.5 mg) was digested with the restriction endonuclease Pstl (20 U) in a 20 µL reaction volume at 37°C for 1 hour. Plasmid pSG311 (2.5 µg) was cleaved with 12 U Smal and 10 U Sstl under identical conditions. The reactions were terminated by addition of 1 volume of phenol: CHCl₃: isoamyl alcohol, precipitated with ethanol, and pelleted for 5 minues at 13,000 x g. The DNA was resuspended in 17.3 µL sterile distilled water. The single stranded 3' overhangs left by Pstl and Sstl were filled-in using T4 DNA polymerase (1.5 U; Bethesda Research Laboratories) in a 20 µL reaction volume containing 20 mM Tris-HCl (pH 7.6), 1 mM MgCl₂, and 0.1 mM dithiotheitol (DTT). After incubating at 15°C for 20 minutes, 2.0 µL of DNA loading buffer was added to the pSG311 sample and the fragment of about 3060 bp isolated using GeneClean from a 0.7% TAE-ethidium bromide-agarose gel after electrophoresis for 1 hour at 100 v. The fill-in reaction with pKK223-3 was stopped by addition of 1 volume phenol: CHCl₃: isoamyl alcohol, precipitated with ethanol, and pelleted at 13,000 x g for 5 minutes. Both DNA samples were resuspended in TE buffer at 100 ng/µL.

A ligation reaction was performed using 100 ng of digested/filled-in pKK223-3 and 325 ng of digested/filled-in pSG311 fragment in a 20 µL volume. After 2 hours at room temperature, 2 µL of the mixture was used to transform competent XL-1 Blue cells, which were spread onto SB plates containing 100 mg/µL of Ampicillin. Very strongly lipolytic colonies appeared after 24 hours incubation at 37°C. Mini-plasmid preparations from three such transforments were analyzed by cleavage with BamHl. The expected fragment in the correct orientation relative to the tac promoter was obtained in all instances. This plasmid was named pSG370.

### 3. Transformation and expression of recombinant Pseudomonas sp. ATCC 21808 lipase in E. coli strains BL21 and W3110

Plasmids pSG312 and pSG370 were transformed into competent E. coli strains BL21 (Studier and Moffett, J. Mo. Biol. 189, 113-130 [1986] and W3110 (Bachmann, Bateriological Reviews 36, 525-542, [1972]), then spread onto SB Cm or SB Ap agar plates. Lipolytic colonies appeared after 24-36 hours at 37°C. The largest zone of activity appeared when pSG312 and pSG370 were transformed into BL21 and W3110, respectively. Plasmids obtained from BL21 (pSG312) and W3110(pSG370) were confirmed to have the expected restriction pattern. For quantitative lipase assays, these strains were first grown in LB Cm or LB Ap at 30°C to OD₆₀₀=0.6. They were frozen in liquid nitrogen following addition of 0.15 ml 100% sterile glycerol (Sigma) to 0.85 ml of culture in a 2 mL cryovial, then stored at -70°C. Cultures for assay were prepared by inoculating 25 mL LB Cm or LB Ap in a 300-µL flask with 1 µL of the thawed vial sample. The flasks were incubated at 30°C, 250 rpm, for 16 hours. The next morning, 2 mL aliquots were withdrawn hourly and stored at 4°C. Lysis was achieved by adding 2.5 µL of a sterile 20% (w/v) sodium dodecylsulfate (SDS) solution to 250 µL of cell culture in a 1.5 mL microfuge tube. Samples were shaken or rotated gently at room temperature for 1 hour, then at 4°C for an additional 16-24 hours. Lysis was complete when the turbid culture cleared and became viscous.

Lipase activity was measured by a pH-stat method in a solution consisting of 300 mL distilled water, 300 mL Sigma Lipase Substrate (Sigma), 120 ml 3.0 mM NaCl, 120 mL 1.5% (W/V) taurocholic acid (Sigma), and 60 mL 0.075 M CaCl₂. One unit (U) is the amount of enzyme which liberates 1 micromole of titratable triglyceride per minute at 37°C, pH 8.5. These results are shown in Table 1.

### D. Stereospecific resolution by transesterification using cloned Pseudomonas sp. ATCC 21808 lipase

SQ35658 is a key chiral intermediate in the total synthesis of R (+) BMY 14802, a new compound being developed for treatment of schizophrenia. The racemic alcohol of the precursor compound SQ35657 was resolved by a enantioselective transesterification in the presence of isopropenyl alcohol and E. coli-derived lipase.

The reaction mixture consisted of 10 mL heptane, 500 mg of racemic alcohol (SQ35657), 500 mg of isopropenyl acetate, and 500 mg of enzyme (4,000 U). The mixture was incubated at 40°C, 250 rpm, in a rotary water bath. After 142 hours, the substrate and product were analyzed by a GC assay. An HP-1 fused silica capillary column (25 m, 0.32 mm internal diameter, 0.17 µm thickness) at 150°C injection temperature, 250°C detector temperature, and 120°C-150°C column temperature (3°C/minute) was used. The total reaction time was 10 minutes. Retention times were 5 and 5.8 minutes for SQ35657 and SQ35658, respectively. A 42% yield of SQ35658 (50% theoretical) was obtained.

The optical purity of the SQ35658 was determined by chiral HPLC using a diode array detection system. A 10 µL aliquot of the reaction mixture was injected into a Bakerbond Chiralcel OB column at 0°C. Chromatography was carried out in a mobile phase consisting of 94% heptane, 5% isopropanol and 1% ethanol. The flow rate was 0.5 mL/minute and the detection wave length was 270 nm. Retention times for the R(+) and S(-) is isomers were 25.4 and 29.8 minutes, respectively. An optical purity of 93% was obtained.

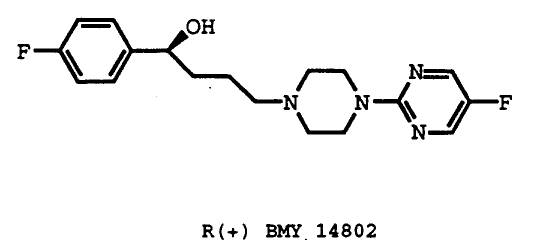

### Resolution of SO 35657 to SO 35658 by Transesterification

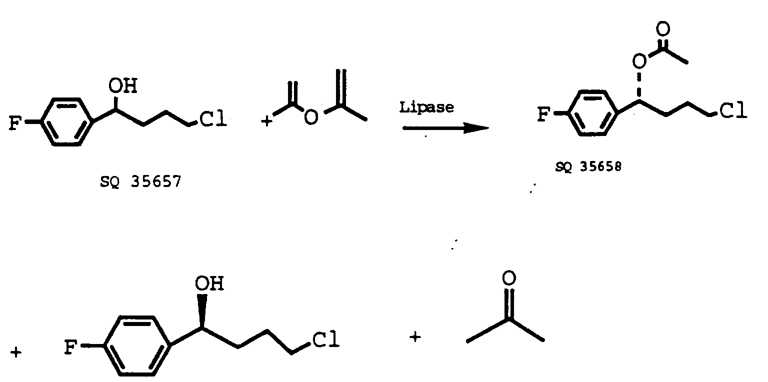

### E. Assymetric hydrolysis of SQ33895 TO SQ34821

SQ34821 is a key chiral intermediate in the total chemical synthesis of SQ30741, a compound under development as a potential thromboxane antagonist. The asymmetric hydrolysis of the diacetate SQ33895 to the chiral monoacetate SQ34821 was carried out by the cloned lipase in a biphasic system.

The reaction mixture contained 27 mL of 50 mM potassium phosphate buffer, pH 7.0, 3 mL toluene, 150 mg SQ33895, and 50 mg (5000 U) of enzyme. The reaction was incubated at 5°C, at 250 rpm, on a rotary shaker. Samples were removed for conversion efficiency and optical purity.

The products of the conversion (1 µL sample) were analyzed by gas chromatography. An HP-5 capillary column (5% diphenyl-95% dimethylopolysiloxane, 25 m x 0.2 mm x 0.11 µm film thickness) was used at 150°C column temperature, 250°C detector temperature, and 250°C injector temperature. A flame ionization detector was used to analyze the eluted compounds. The retention times for SQ33895 and SQ34821 were 6.00 and 9.13 minutes, respectively. The yield of SQ34831 was 70 M %.

Optical purity of SQ34821 was determined by chiral HPLC. A Chiracel OB column (25 cm x 4 mm) was used in a Hewitt-Packard 1090 HPLC apparatus. The mobile phase was heptane: isopropanol (70:30) at a flow rate of 0.5 mL/minute. Detection was carried out at a wavelength of 220 nm. The retention times for the (+) and (-) enantiomers of SQ34821 were 8.5 and 11.4 minutes, repectively.

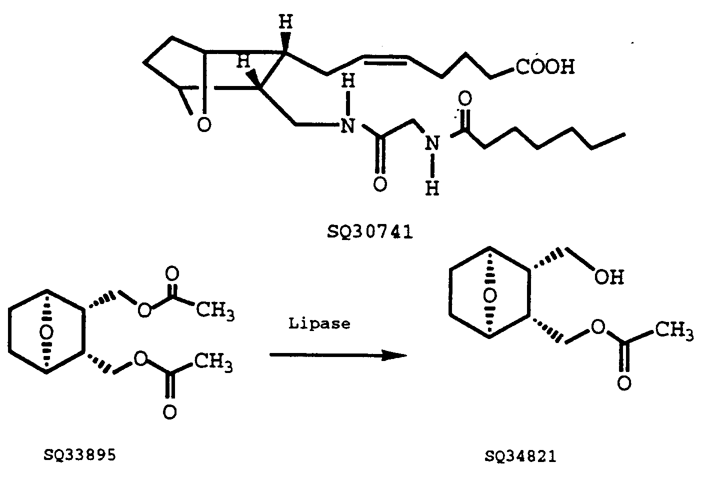

**Table 1**

| **Lipase Activity of Recombinant E. coli Strains Possessing the Cloned Pseudomonas sp ATCC 21808 Lipase Gene** | |
|---|---|
| **Strain** | **Peak Lipase Activity (U/ml lysate)** |
| BL21 | <2.0 U/mL |
| BL21 (pBC-SK⁺) | <2.0 U/mL |
| BL21 (pSG312) | 71.5 U/mL |
| W3110 | <2.0 U/mL |
| W3110 (pKK223-3) | <2.0 U/mL |
| W3110 (pSG370) | 95.2 U/mL |

The supernatants of the above recombinant strains demonstrated no lipase activity.

## Claims

1. An isolated nucleic acid molecule comprising a nucleic acid sequence coding for a lipase of Pseudomonas sp. ATCC 21808, wherein said lipase has the amino acid sequence encoded by the nucleotide sequence of the Sacl/Hindlll insert of plasmid pSG312.

2. The nucleic acid molecule according to Claim 1 which is a DNA molecule and wherein the nucleic acid sequence is a DNA sequence.

3. A DNA molecule having a DNA sequence which is complementary to the DNA sequence according to Claim 2.

4. An expression vector comprising a DNA sequence coding for a lipase of Pseudomonas sp. ATCC 21808, wherein said lipase has the amino acid sequence encoded by the nucleotide sequence of the Sacl/Hindlll insert of plasmid pSG312.

5. A prokaryotic or eukaryotic host cell containing the expression vector according to Claim 4.

6. A method for producing a polypeptide molecule which comprises a lipase of Pseudomonas sp. ATCC 21808, wherein said lipase has the amino acid sequence encoded by the nucleotide sequence of the Sacl/Hindlll insert of plasmid pSG312 comprising culturing a host cell according to Claim 5 under conditions permitting expression of the polypeptide.

7. An isolated polypeptide molecule comprising a recombinant lipase of Pseudomonas sp. ATCC 21808, wherein said lipase has the amino acid sequence encoded by the nucleotide sequence of the Sacl/Hindlll insert of plasmid pSG312.
